(19) Europäisches Patentamt
European Patent Office
Office européen des brevets

(11) Publication number: 0 458 751 A1

# (12) EUROPEAN PATENT APPLICATION

(21) Application number: 91810380.5

(22) Date of filing: 17.05.91

(51) Int. Cl.$^5$: A61K 31/195, A61K 9/54, A61K 31/215

(30) Priority: 25.05.90 US 530768

(43) Date of publication of application: 27.11.91 Bulletin 91/48

(84) Designated Contracting States: BE CH DE DK ES FR GB GR IT LI NL SE

(71) Applicant: WARNER-LAMBERT COMPANY
201 Tabor Road
Morris Plains New Jersey 07950 (US)

(72) Inventor: Cherukuri, Subraman Rao
10 Jean Drive
Towaco, New Jersey 07082 (US)
Inventor: Chau, Tommy Linkwong
3 Dartmouth Avenue-3A
Bridgewater, New JErsey 08807 (US)

(74) Representative: Silbiger, Jakob, Dr.
c/o CAPSUGEL AG Fabrikmattenweg 2-4
CH-4144 Arlesheim (CH)

## (54) Delivery system for cyclic amino acids with improved taste, texture and compressibility.

(57) A new delivery system for cyclic amino acid compounds and the process for its preparation is disclosed, which has use in a variety of products including comestibles such as chewing gum compositions, confections, and pharmaceutical such as chewable tablets. More particularly, this invention relates to a process for preparing a delivery system that provides enhanced masking of the bitter flavour characteristic of the active, reduced grittiness, retained stability at the elevated temperatures of product formulation, and improved compressibility for formation into tablets.

EP 0 458 751 A1

The present invention relates generally to cyclic amino acids that are useful as medicaments, and particularly to the preparation of such cyclic amino acids in a form to reduce their inherent bitterness and grittiness.

Certain cyclic amino acid medicaments, commercially known as GABAPENTIN® and manufactured by Goedecke of West Germany, a company of Warner-Lambert Company of Morris Plains, New Jersey, are used in the treatment of certain cerebral diseases, and for example, are prescribed for the treatment of certain forms of epilepsy, faintness attacks, hypokinesia and cranial traumas. They are also noted to improve cerebral functions and are therefore useful for the treatment of geriatric patients. The cyclic amino acids are prepared in a variety of compounds including inorganic salts such as the halides, nitrates and sulfates, and organic compounds including the acetates, citrates, gluconates, and compounds with various amino acids and vitamins. These compounds have been formulated into solid and liquid compositions for a variety of hygienic and nutritional purposes and presently enjoy particular popularity and value in such connection.

The cyclic amino acids and their preparation is taught in U.S. Patent No. 4,024,175, issued May 17, 1977 to Satzinger et al. assigned to Warner-Lambert Company. U.S. Patent No. 4,152,326 to Hartenstein et al., issued May 1, 1979 and assigned to Warner-Lambert Company, discloses an alternative synthetic route for the preparation of the active compounds and covers particular cyclic sulphonyloxyimides that serve as reactive intermediates in the preparation of such active compounds. The disclosures of both patents are incorporated herein by reference.

One of the major drawbacks of the incorporation of these cyclic amino acids into various orally received products has been a characteristic bitterness that is experienced as soon as the active compound is released and proceeds to break down in the mouth. A variety of formulations have been prepared and attempted for the purpose of lessening or masking entirely the bitter aftertaste of the active compound. This problem is particularly acute, as these compounds are extremely watersoluble and readily escape from any taste-masking complexes into which they may be formulated.

Thus, the active has been disposed in a variety of media including combination with flavours and with other compounds in an attempt to achieve a neutralizing or masking effect on the bitter aftertaste. However, none of the presently known techniques and compositions appear to offer the desired abatement of bitter aftertaste.

A further problem that is encountered with these active compounds is that it is difficult to prepare a compressible formulation such as for the preparation of tablets. The active compounds as prepared are usually recovered as a fine powder that resists preparation into tablets by conventional compression forming. The tablets tend to either crumble in the die or disintegrate prematurely during shelf storage. This deficiency is not remedied merely by pre-coating the active, as the coating materials themselves are somewhat brittle and the resulting material frequently cracks during the forming process.

More generally, the encapsulation of actives is well-known for a variety of purposes, among them to protect the active from degradation in contact with other agents in a given product or composition; to modulate the release of the active, as in the instance of flavour and sweeteners, and to render the active capable of withstanding rigorous processing conditions during formulation into products. Efforts have also been made in various instances to render actives more palatable likewise by enrobing or encapsulating the active in one fashion or another. The following representative techniques are noted in the art with respect to differing actives.

U.S. Patent No. 4,384,004 to Cea et al. discloses solid particles of aspartame encapsulated by a coating material selected from the group consisting of cellulose, cellulose derivatives, arabinogalactin, gum arabic, polyolefins, waxes, vinyl polymers, gelatin, zein and mixtures thereof, wherein the amount of said coating material to said methyl ester is from 1:1 to 1:10. The objective of this invention is to provide protection to the active and to delay its release.

U.S. Patent No. 3,867,556 to Darragh et al. also encapsulates volatile flavours in a fat or wax material. The patentees had found that the fat/wax encapsulation displayed excessive instability to heat, and as their product was intended primarily for incorporation into baked goods, they applied a second coating of a water-soluble material such as gum arabic, which would provide high temperature stability while conferring rapid disintegration on contact with moisture.

Efforts to protect and mask therapeutic actives are disclosed in the prior art. Accordingly, in Canadian Patent No. 1,234,761 to Lapidus, a chewable buffered aspirin mixture is prepared into a tablet form by combination with a fatty material, whereby the aspirin and buffering materials may be individually coated and thereby prevented from cross reaction with each other. The Lapidus disclosure concerns itself with the problems of the grittiness and undesirable taste sensation that is experienced with the pharmaceutical actives, and proposes that the invention cures these deficiencies as well. The patent contains a review of patent literature relevant to the treatment and tabletting of aspirin, and indicates that such approaches as inclusion of flavourings and the sequential and separate addition to a capsule product of the aspirin and the alkaline buffering component are known.

European Patent Publication No. 0266113 based on Application No. 87309255.5 to Blank et al. and assigned to American Home Products Corporation discloses a preparation of spray dried acetaminophen prepared in a solution of a cationic copolymer based on dimethyl aminoethyl methacrylate and neutral methacrylic acid esters. The Blank et al. formulation is indicated to be "taste-neutral" and "fast dissolving." The specific co-polymer is a member of a group of coating compositions that are sold under the name "EUDRAGIT" by Rohm Tech Incorporated, the United States representative of Rohm Pharma GmbH of West Germany. As mentioned, however, the use of this copolymer alone results in particles which will fracture under tabletting compression owing to the hardness of the copolymer coating material.

European Patent Publication No. 0212641 based on Application No. 86111636.6 to Damani et al. and assigned to G. D. Searle & Co. relates to taste masking compositions wherein the active is incorporated into a copolymer having a plurality of carboxylic acid and ester groups. The formulation of the matrix of the active and the copolymer takes place with a solvent which is thereafter removed to yield a porous drugpolymer matrix. One of the features of this particular formulation is that the active will be released from the matrix at an acidic pH and, in particular, a pH of less than 4. In this instance, anionic copolymers are utilized and a variety of such copolymers including acrylic and substituted acrylic acids, cellulose esters, vinyl and substituted vinyl esters and polysulfonic acids and esters and amides are included. The polymers are particularly well-suited for actives having amido functional groups, such as alkaloids, amines, amides, and the like. One of the purported advantages of the formulations disclosed in this patent is indicated to be the taste masking abilities of the preparation.

European Patent Publication No. 0265226 based on Application No. 87309253.0 by Blank et al. and assigned to American Home Products Corporation discloses a preparation of a spray dried and neutral tasting acetaminophen powder using a solution of ethylcellulose with a suspension of colloidal silica in a lower alkanol solvent.

Lastly, PCT International Publication No. WO 88/03795 based upon Application No. PCT/US/87/03068 by Mehta, reveals a taste-masked composition coated with a combination of low temperature and high temperature film-forming materials such as, for example, cellulose esters, vinyl polymers, copolymers of methacrylic acid, and copolymers of methacrylic acid esters. The latter two mentioned polymeric materials are also members of the "EUDRAGIT" family of coating compositions.

All of the foregoing formulations, however, do not provide the desired level of taste masking and the combination of these qualities with compressibility in the instance where tablets are to be formed, particularly with respect to the cyclic amino acid actives that are presently of particular interest herein.

The above noted and other known techniques have heretofore provided no insight into the problem of concealing the bitter aftertaste of the present active, while at the same time permitting the active to exhibit improved compressibility in the instance where it is to be prepared in tablet form, as well as to function in a timely manner and with improved mouthfeel in the products to which it is desirably added. A need therefore exists for the development of a delivery system which remedies the aforenoted problems by preventing or at least minimizing the bitterness of the compound while at the same time permitting timely release of the active and promoting improved textural and mechanical properties.

According to the present invention there is provided a delivery system for a cyclic amino acid compound providing reduced bitterness with improved mouthfeel, compressibility, and high temperature stability, comprising:

(a) a core material comprising a cyclic amino acid compound;

(b) a first polymeric coating selected from waterinsoluble and water-soluble polymeric film-forming materials, in an amount of from about 5% to about 100% by dry weight of the core material; and

(c) a second hydrophilic coating selected from the group consisting of fats, fatty acids, waxes and mixtures thereof, present in an amount ranging from about 20% to about 400% by dry weight of the combination of said core material and said first hydrophilic coating.

The present invention provides a cyclic amino acid compound delivery system which comprises a composite having improved flavour masking and temperature stability characteristics, and smoother texture and mouthfeel when incorporated into lozenges, chewing gums and other products, and improved compressibility for preparation into products. The compound delivery system is prepared with a core material comprising a cyclic amino acid compound; a first coating selected from water-soluble or water-insoluble polymeric film-forming materials in an amount of from about 5% to about 100% by dry weight, and preferably from about 10% to about 25% by dry weight of the core material; and a second hydrophobic coating comprising a fat and/or wax component in an amount of from about 20% to about 400% by dry weight, and preferably from about 20% to about 100% by dry weight of the combined core material and first coating. In a particularly preferred embodiment, the first coating is present in an amount of about 25% by dry weight of the core material, and the second coating is present in an amount of 50% by dry weight of the combined core material and first coating.

Suitable cyclic amino acid compounds may be selected from the group consisting of compounds of the general formula:

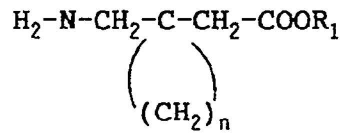

wherein $R_1$ is a hydrogen atom or a lower alkyl radical and n is 4, 5 or 6; and the pharmacologically compatible salt thereof. The first coating includes both water-soluble and water-insoluble polymeric materials, with the water-insoluble materials including acrylic acid and substituted acrylic acid polymers and copolymers; vinyl and substituted vinyl ester polymers and copolymers; cellulose ethers, their polymers and copolymers; and polysulfonic acids, their esters and/or amides. The first coating may also be a water-soluble polymeric material such as a hydrocolloid. Hydrocolloid materials include pectins, alginates, cellulose and its derivatives, gelatin, gums, mucilages, and mixtures. The gelatin used herein possesses a bloom strength on the order of 250 or higher, which is desirable to form a strong and resilient coating on the active.

The fat or wax component comprises fats, including fatty acids such as hydrogenated and partially hydrogenated oils; mono-, di- and triglycerides, polyglycerol esters and sorbitol esters. Waxes include natural and synthetic waxes, with representative waxes comprising polyolefin waxes, paraffin wax, beeswax, microcrystalline wax, and mixtures.

The cyclic amino acid compounds are prepared into the core material by standard processes such as spray drying and may optionally be prepared with additives such excipients, including bulking agents, fillers, and the like. Suitable excipients include sugar, mannitol, sorbitol and maltodextrin.

The present delivery system is produced by a process which comprises forming the cyclic amino acid compound and optionally one or more excipients into a first core material, applying the first coating to this core material preferably by fluidized bed coating, and then spray congealing the second fat and/or wax coating over the particles thus formed.

The present delivery system may be incorporated into a variety of foods and confections, including chewing gums and hard candies, as well as pharmaceutical and preparations, such as chewable tablets. The present invention therefore includes chewing gums, hard candies and pharmaceutical products, all incorporating the present delivery system.

Accordingly, it is a principal object of the present invention to provide a cyclic amino acid compound delivery system that offers improved aftertaste masking characteristics.

It is a further object of the present invention to provide a cyclic amino acid compound delivery system as aforesaid which provides improved taste masking and mouthfeel in combination with temperature stability, and improved compressibility.

It is a still further object of the present invention to provide,,pharmaceutical products, nutritional supplements, personal hygiene, confectionery and comestible products, all having contained therein the cyclic amino acid compound delivery system of the present invention.

Other objects and advantages will become apparent to those skilled in the art from a consideration of the ensuing description.

In accordance with the present invention, a delivery system for a cyclic amino acid compound is disclosed which offers reduced bitterness, and improved mouthfeel with desirable high temperature stability. The present delivery system accordingly comprises:

(a) a core material comprising a cyclic amino acid compound;

(b) a first polymeric coating selected from waterinsoluble materials in an amount of from about 5% to about 100% by dry weight of the core material; and

(c) a second hydrophobic coating selected from the group consisting of fats, waxes, and mixtures thereof, present in an amount of from about 20% to about 400% by dry weight of the combination of the core material and the first hydrophilic coating.

More particularly, the delivery system of the present invention comprises the core material with the first coating present in an amount of from about 10% to about 25% by dry weight of the core material, and the second coating present in an amount of from about 20% to about 100% by dry weight of the combination of the core material and the first coating. As indicated earlier herein, the preferred embodiment contemplates the presence of the first coating in an amount of 25% by dry weight of the core material, and the second coating in an amount of 50% by dry weight of the combination of the core material and the first coating.

The cyclic amino acid compounds in accordance with the present invention comprise those compounds having the following formula:

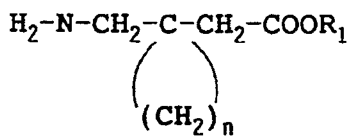

wherein $R_1$ is a hydrogen atom or a lower alkyl radical and n is 4, 5 or 6; and the pharmacologically compatible salts thereof.

The preparation and utility of those compounds are set forth in commonly assigned U.S. Patent No. 4,024,175 to Satzinger et al., and U.S. Patent No. 4,152,326 to Hartenstein et al. Both patent disclosures are incorporated herein by reference.

Particular cyclic amino acids include 1-aminomethyl-1-cyclohexane-acetic acid; ethyl-1-aminomethyl-1-cyclohexane-acetate; 1aminomethyl-1-cycloheptane-acetic acid; 1-aminomethyl-1-cyclopentane-acetic acid; methyl 1-aminomethyl-1-cyclohexane-acetate; n-butyl 1-aminomethyl-1-cyclohexaneacetate; methyl 1-aminomethyl-1-cyclohexane-acetate; n-butyl 1-aminomethyl-1-cycloheptane acetate toluene sulfonate; 1-aminomethyl-1-cyclopentane-acetate benzene-sulfonate; and n-butyl 1-aminomethyl-1-cyclopentane-acetate. The foregoing list is not meant to be inclusive, and is merely representative of cyclic amino acid compounds that may be prepared into the delivery system of the present invention.

The cyclic amino acid compounds may optionally be formulated with one or more excipients. Such excipients may be selected from the group consisting of carbohydrate materials, polyhydric alcohols and mixtures thereof. Carbohydrates useful as excipients include traditional water-soluble sweetening agents such as monosaccharides, disaccharides and polysaccharides such as xylose, ribose, glucose, lactose, mannose, galactose, fructose, dextrose, sucrose, sugar, maltose, partially hydrolysed starch, or corn syrup solids and sugar alcohols such as sorbitol, xylitol, mannitol and the like, and mixtures thereof. The excipients are generally present in amounts of up to about 80% by dry weight of the core material and can be mixed in combination with each other or used individually.

Suitable water-insoluble film-forming polymeric materials are selected from the group consisting of acrylic acid and substituted acrylic acid polymers and copolymers; polysulfonic acid ester polymers; vinyl and substituted vinyl ester polymers and copolymers; cellulose ethers, their polymers and copolymers. Particular water-insoluble film-forming polymeric materials are selected from the group consisting of ethylcellulose; cellulose acetate; cellulose acetate phthalate; cellulose acetate butyrate; ethylene-vinyl acetates and/or phthalates; polyvinyl alcohol; polyvinyl acetates and/or phthalates; ethyl and/or methyl methacrylic acids, esters, and copolymers. Particularly preferred materials comprise ethylcellulose; the anionic copolymers based on polymethacrylic and acrylic acid esters; the neutral copolymer based on poly(meth)acrylic acid esters; and the cationic copolymer based on dimethylamino ethyl methacrylate and neutral methacrylic acid esters. The three acrylic-based polymers are sold under the trade name EUDRAGIT by Rohm Pharma of West Germany, and the ethylcellulose is sold as an aqueous suspension under the trade name AQUACOAT® by FMC, of Philadelphia, Pennsylvania.

Water-soluble polymeric materials suitable for preparation of the first coating broadly comprise hydrophilic materials and particularly, film forming hydrocolloids. The hydrocolloids May be generally Selected from the group consisting of gums, pectins, alginates, mucilages, and mixtures thereof. Specifically, the hydrocolloid may be a material selected from the group consisting of gum arabic, tragacanth, karaya, ghatti, agar, alginates, carrageenans, fucellan, psyllium, and mixtures thereof. The hydrocolloid may also be selected from polyvinyl pyrrolidone, gelatin, dextran, xanthan, curdan, cellulose, methylcellulose, ethylcellulose, hydroxyethyl cellulose, hydroxypropyl cellulose, hydroxypropyl methylcellulose, carboxymethyl cellulose, low methoxy pectin, propylene glycol alginate, and mixtures thereof. A preferred hydrocolloid comprises gelatin.

Materials suitable for the preparation of the second coating include the fats and/or waxes. Suitable fats include fatty acids such as hydrogenated or partially hydrogenated oils, with representative materials comprising palm oil, palm kernel oil, soybean oil, cottonseed oil, peanut oil, rapeseed oil, rice bran oil, sunflower oil, safflower oil, and mixtures thereof. Other materials also useful as fats herein may be selected from monoglycerides, diglycerides, triglycerides, polyglycerol esters, sorbitol esters, and mixtures thereof.

Suitable waxes include natural waxes, synthetic waxes, and mixtures thereof, and in particular, comprise materials selected from the group consisting of paraffin wax, beeswax, carnauba wax, candelilla wax, lanolin wax, bayberry wax, sugar cane wax, petrolatum, carbowax, spermaceti wax, rice bran wax, microcrystalline wax, and mixtures thereof. Naturally the foregoing is illustrative and not restrictive of suitable materials for inclusion in the delivery system of the invention, and the invention is considered to extend to unnamed equivalent materials within its scope.

The preparation of the present delivery system may be accomplished by a variety of agglomerative and/or

coating techniques known in the art including, spray drying, fluidized bed coating techniques and the like, as disclosed in U.S. Patent No. 4,384,004 to Cea et al. Preferably, fluidized bed coating may be employed to form the initial core as well as to apply the first and second coatings. In the fluidized bed procedure as applied herein, initially liquid droplets and subsequently, particles of the core material are suspended in an apparatus that creates a strong upward air current or stream in which the particles move. The stream passes through a zone of finely atomized coating material which causes the passing particles to be coated, after which the coated particles move from the upward stream and travel downward in a fluidized condition countercurrent to a flow of heated fluidized gas whereupon they are dried. The particles may reenter the upward stream for a further coating or may be withdrawn from the coating apparatus. The foregoing method and apparatus are known as the Wurster Process and are set forth in detail in the following U.S. Patents, the disclosures of which are incorporated herein by reference: U.S. Patent No. 3,089,824; U.S. Patent No. 3,117,027; U.S. Patent No. 3,196,827; U.S. Patent No. 3,241,520; and U.S. Patent No. 3,253,944.

The first coating material is prepared for use by the formation of a liquid capable of being uniformly atomized. Thus, the water-insoluble polymeric materials may be prepared as dispersions, and the water-soluble hydrocolloid materials may be prepared as aqueous solutions. If desired, other ingredients such as plasticizers may be added to improve the properties of the final coating. Suitable plasticizers include the glyceryl ester of sebacic acid, dibutyl sebacate, diethyl phthalate, glyceryl triacetate, tributyl citrate, acetylated monoglyceride, citric acid ester of monodiglyceride, adipate ester, and others. The plasticizers may be added in known effective amounts within the scope of the invention.

While the second hydrophobic coating is preferably applied to the core material coated with the first coating by a fluidized bed coating technique as described above, the technique known as spray congealing may also be used. In this technique, the fat or wax material is heated to a temperature of about 75° to about 95°C and placed under low shear mixing, after which the core material coated with the first coating is added and mixed at high shear to achieve a uniform dispersion of the core materials therein. The dispersion is then fed into a heat controlled spray nozzle and is spray congealed. The term "spray congealing" as used herein refers to the solidification of the atomized liquid droplets which cool and solidify upon hitting the cooler temperature of the surrounding atmosphere, which may, for example, be on the order of 25°C. The nozzle pressure is regulated to control particle droplet size, and droplets cool and congeal once they are emitted from the nozzle and contact the cooler environment. The result of this process is a dry particle or agglomerate which may have an approximate elliptical or spherical shape.

The resultant product of this invention is in powder or granulated form. The particle size is not critical to the delivery system and can be adjusted to accommodate a particular desired release rate and mouthfeel, depending on the vehicle, e.g., chewing gum, confection or pharmaceutical preparation in which it is incorporated. The core material can include a wide variety of materials such as sweeteners, medicaments, drugs, flavouring agents, and the like in addition to the desired active compounds. These materials can be used either singly or in combination in either a single or multiple delivery system. That is, one or more of these materials may be present within one coating matrix or separately coated by the matrix and employed alone or in combination in a final product. Desirably, the resultant product should contain a high loading of the cyclic amino acid active, and accordingly, active contents on the order of up to about 10% or higher-based or dry weight are contemplated herein.

The instant delivery system can be incorporated in a number of ingestible products such as confections and the like, as well as chewing gum compositions and pharmaceutical preparations.

As indicated herein, the delivery system of the present invention may be incorporated into a variety of hard candies such as lozenges and the like. Such candies may be prepared by procedures as well-known in the art. Thus, a candy base which may comprise a sugar or sugar alcohol such as sucrose, fructose, glucose, lactose, polydextrose, mannitol, sorbitol, maltitol, corn syrup, hydrolysed starch and the like, is subjected to heating at temperatures ranging up to around 250°F to 300°F in an open kettle, during which time additives such as flavour and colorants are included. Likewise, the delivery system of the present invention may then be incorporated therein. After all ingredients are included, the resulting candy base is processed by a technique known as "pulling" which constitutes the literal hand stretching of the candy base mass to uniformly blend all of the ingredients therein. After "pulling" is complete, the resulting mass may be laid flat or otherwise disposed on a flat sheet and allowed to cool, during which time individual portions may be prepared. Naturally, the foregoing general description of hard candy manufacture is merely exemplary and presented herein for purposes of describing a best mode for carrying out one embodiment of the invention.

As noted earlier, the present delivery system may be prepared into tablet form, and may accordingly be formulated with known tabletting additives, such as excipients, and the like. The present delivery system displays improved compressibility, and tablets formed therefrom exhibit integrity and shelf life.

Suitable excipients include disintegrating agents, lubricating agents, glidants, binders and diluents. Disintegrants may be selected from a wide variety of agents, and preferable are either swelling or wicking types. Exemplary disintegrating agents, which normally are materials that tend to swell in the presence of water or draw in water and thus facilitate tablet wetting, include carboxymethyl starches; clays such as Veegum HV and bentonite; celluloses such as purified cellulose, sodium carboxymethylcellulose and carboxymethylcellulose and cross-linked derivatives thereof as well as microcrystalline cellulose; and crosslinked polyvinylpyrrolidone. The amount of disintegrating agent may vary broadly and is preferably from about 1% to about 50% by dry weight of the tablet.

Lubricants are used in the tablet reagent in order to ease the ejection of the tablet from the die, to prevent sticking of the tablets to the punches and to limit wear on dies and punches. Lubricants may be selected from a wide range of materials which are both water and organic solvent soluble as well as finely divided particles such as calcium stearate, magnesium stearate, zinc stearate, stearic acid, hydrogenated vegetable oils, talc, light mineral oil, sodium benzoate, sodium lauryl sulfate, magnesium lauryl sulfate, polymeric fluorocarbons and mixtures thereof. The polymeric fluorocarbons may be selected from a well-known group of polymeric and copolymeric substances made up of carbon and fluorine, which, in addition, may contain hydrogen and/or chlorine. The fluorocarbons may include at least one fluoroolefin; for example, polytetrafluoroethylene, and copolymers of tetrafluoroethylene and hexafluoropropylene are contemplated. Also included would be polyvinylidene fluoride, a copolymer of vinylidene fluoride and hexafluoropropylene, as well as other polymeric fluorocarbons recited in U.S. Patent No. 4,405,486 to Eoga. Polymeric fluorocarbons are the preferred lubricant in view of its ready availability and efficient lubrication properties.

The lubricants which are not jointly soluble should be in as fine a state of subdivision as possible because the smaller the particle size the greater the efficiency in the granulation or powder mix. Preferred sizes are those that pass through an 80 or 100 mesh screen (U.S. Standard Mesh screen) and most preferred through a 200 mesh screen before use. The amount of lubricant will vary broadly and is preferably from about 0.1% to about 5% by dry weight of the total composition.

Glidants are used to improve the flow of granulations from hoppers into feed mechanisms and ultimately into the tabletting dye cavity. They often minimize the degree of surging occurring during direct compression and act to minimize the tendency of a granulation to separate or segregate due to excessive vibration. Glidants may be selected from a wide range of materials which are preferably water-soluble or organic solvent soluble and, if not jointly soluble, should preferably be in as fine a state of subdivision as possible. Preferred sizes are those that pass through an 80 or 100 mesh screen (U.S. Standard Mesh size). Exemplary materials include talc, cornstarch, colloidal silicon dioxides and silicas.

Binders that are used when a wet granulation process is employed include starch, pregelatinized starch, gelatin, free polyvinylpyrrolidone, methylcellulose, sodium carboxymethylcellulose, polyvinylalcohols and so forth. Binders when used can be employed in amounts up to about 25% and preferably about 5% to about 15% by dry weight.

Diluents when used are selected to provide effective physicochemical changes to improve processing or manufacture as well as stability of tablet configuration. Diluents aid in providing a degree of cohesiveness when tablet size and rate of dissolution are important. Suitable diluents useful in the tablets of this invention include carbohydrates such as starch, dextrose and sucrose and polyhydric alcohols such as sorbitol, mannitol, xylitol, pentaerythritol and polymers thereof such as polyethers, nitrogen containing compounds such as urea and alkylureas and mixtures thereof. These materials may be used in amounts of about 40% to about 97% by dry weight of the tablet.

The pressed tablets are prepared by conventional means using standard techniques and equipment known to those skilled in the art. Preferred procedures of preparing the tablets of this invention involve the direct compression method or wet granulation method.

In a typical direct compression method, the indicator composition in the form of a particulate solid is blended with the tablet formulation ingredients. Once incorporated, mixing is continued until a uniform mixture is obtained and thereafter the mixture is formed into suitable shapes by subjecting the formulation to a tabletting operation. Compression pressures on the order of up to one-half to 12 tons per square inch are normally employed.

In contrast, a typical wet granulation process for preparing a tablet involves milling of indicator and excipients, mixing of milled powders, preparation of binder solution, mixing of binder solution with powder mixture to form wet mass, coarse screening of wet mass using 6 to 12 mesh screen, drying of moist granules, screening of dry granules through 14 to 20 mesh screen, mixing of screened granules with lubricant and disintegrant and finally tablet compression. Variations hereof may be employed to insure compression of a viable tablet.

The following examples serve to provide further appreciation of the invention but are not meant in any way to restrict the effective scope of the invention. All percentages throughout the Specification are by weight percent of the final delivery system unless otherwise indicated.

EXAMPLE 1

In this example, a coated GABAPENTIN® product was prepared. Accordingly, a quantity of AQUACOAT® ECD-30 having a solid latex of 29.4% with 25.8% ethylcellulose, 1.0% sodium lauryl sulfate and 2.6% cetyl alcohol was admixed with a quantity of dibutyl sebacate and mixed for 15 minutes. Deionized water was thereafter added to this dispersion mixture, and mixing was continued for a further ten minutes. The unmilled GABAPENTIN® was then placed into a Glatt fluid bed agglomerator/dryer and was thereafter coated with the dispersion under the following conditions: coating solution temperature of 27°C; atomizing nozzle diameter of 1.4 mm; spray rate of 7-9 ml/minute; inlet air temperature of 81-97°C; atomizing air pressure of 30 psi; and atomization air temperature of 77-82°C. The filter bag was also shaken at intervals of 2 seconds, and the duration of each shake was 6 seconds. The material that was received was off-white in colour and granular in appearance.

The ingredients and their amounts are set forth in Table I, below.

TABLE I

| Ingredient | Percent w/w | Weight/ Grams |
|---|---|---|
| Unmilled GABAPENTIN® | 28.57 | 600.00 |
| Ethylcellulose-AQUACOAT® ECD-30 | 38.87 | 816.30 |
| Dibutyl sebacate-UNIFLEX DBS | 2.86 | 60.00 |
| Deionized Water | 29.70 | 623.70 |
| TOTAL | 100.00 | 2100.00 |
| Dried Encapsulation | | |
| Unmilled GABAPENTIN® | 66.67 | 600.00 |
| Ethylcellulose-AQUACOAT® ECD-30 | 26.67 | 240.00 |
| Dibutyl sebacate-UNIFLEX DBS | 6.66 | 60.00 |
| TOTAL | 100.00 | 900.00 |

The material prepared above was then subjected to the application of the second hydrophobic coating. In this instance, a quantity of partially hydrogenated soybean oil and glycerol monostearate were mixed and melted and brought to a temperature of 90-95°C. The encapsulated material prepared above was then added to the fluid bed agglomerator dryer utilized above and was coated with the hydrophobic coating material as prepared, under the following conditions: The coating solution temperature was 90-96°C; the atomization nozzle diameter was 1.4 mm; the spray rate was 9 ml/minute; the inlet air temperature was 40-43°C; the atomizing air pressure was 20 psi; the atomization air temperature was 84-89°C; the filter bag was shaken at an interval of 2 seconds and for a duration of 6 seconds. The resulting material appeared white and granular and was ready for storage and/or incorporation into further products.

The ingredients utilized in the final preparation of the product are set forth in Table II, below:

TABLE II

| Ingredient | Percent w/w | Weight/ Grams |
|---|---|---|
| GABAPENTIN® Encapsulation | 66.67 | 400.00 |
| Partially Hydrogenated Soybean Oil | 31.67 | 190.30 |
| Glycerol Monostearate | 1.66 | 10.00 |
| TOTAL | 100.00 | 600.00 |

EXAMPLE 2

A similar particulate product was prepared using the same procedure set forth in Example 1, above. In this instance, the first coating was 250 bloom gelatin type A, which was applied to the unmilled GABAPENTIN® to form the first coated core material. This material was then coated with a mixture of partially hydrogenated soybean oil and glycerol monostearate in the same fashion as in Example 1, and a similar product was obtained. The specific ingredients and their proportions for the preparation of the first and second coatings, respectively, are set forth in Tables III and IV, below.

TABLE III

| Ingredient | Percent w/w | Weight/ Grams |
|---|---|---|
| Unmilled GABAPENTIN® | 33.33 | 700.00 |
| 250 Bloom Gelatin Type A | 6.67 | 140.00 |
| Deionized Water | 60.00 | 1260.00 |
| TOTAL | 100.00 | 2100.00 |
| Dried Encapsulation | | |
| Unmilled GABAPENTIN® | 83.33 | 700.00 |
| 250 Bloom Gelatin Type A | 16.67 | 140.00 |
| TOTAL | 100.00 | 840.00 |

TABLE IV

| Ingredient | Percent w/w | Weight/ Grams |
|---|---|---|
| GABAPENTIN® Encapsulation | 57.14 | 350.00 |
| Partially Hydrogenated Soybean Oil | 40.72 | 249.38 |
| Glycerol Monostearate | 2.14 | 13.12 |
| TOTAL | 100.00 | 612.50 |

EXAMPLE 3

In this example, compressed chewable tablet formulations were prepared and formed by direct compression. The resulting tablets had the ingredients set forth in Table V, below.

TABLE V

| Ingredient | Control (%) | Invention (%) |
|---|---|---|
| GABAPENTIN® | 6.26 | -- |
| Aspartame | 0.63 | 0.63 |
| Sugar | 90.61 | 82.81 |
| Sodium Stearyl Fumarate | 1.00 | 1.00 |
| Spray Dried Cream Strawberry Flavour | 1.00 | 1.00 |
| Dilute Monoammonium Glycyrrhizinate | 0.50 | 0.50 |
| Encapsulation (AQUACOAT®/Fat) | -- | 14.06 |

The tablets were then subjected to taste panel testing and were evaluated for bitterness. The scale utilized extended from 0, reflecting no bitterness, to 100, indicating extreme bitterness. The control composition containing free GABAPENTIN® exhibited extreme bitterness, while the encapsulated composition prepared in accordance with the present invention had a rating of 40, indicating substantially reduced bitterness.

This invention may be embodied in other forms or carried out in other ways without departing from the spirit or essential characteristics thereof. The present disclosure is therefore to be considered as in all respects illustrative and not restrictive, the scope of the invention being indicated by the appended Claims, and all changes which come within the meaning and range of equivalency are intended to be embraced therein.

Having been thus described, the present invention is summarised by the following clauses, numbered 1 - 28, and is defined by the claims appended thereafter.

1. A delivery system for a cyclic amino acid compound providing reduced bittemess with improved mouthfeel, compressibility, and high temperature stability, comprising:
   (a) a core material comprising a cyclic amino acid compound;
   (b) a first polymeric coating selected from waterinsoluble and water-soluble polymeric film-forming materials, in an amount of from about 5% to about 100% by dry weight of the core material; and
   (c) a second hydrophilic coating selected from the group consisting of fats, fatty acids, waxes and mixtures thereof, present in an amount ranging from about 20% to about 400% by dry weight of the combination of said core material and said first hydrophilic coating.
2. The delivery system of 1 wherein said cyclic amino acid compound has the formula:

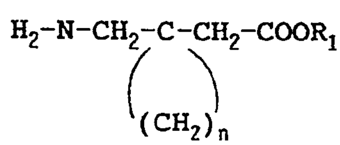

wherein $R_1$ is a hydrogen atom or a lower alkyl radical and n is 4, 5 or 6; and the pharmacologically compatible salt thereof.

3. The delivery system of 1 wherein said cyclic amino acid compound is selected from the group consisting of 1-aminomethyl-1-cyclohexane-acetic acid; ethyl-1-aminomethyl-1-cyclohexane-acetate; 1-aminomethyl-1-cycloheptane-acetic acid; 1-aminomethyl-1-cyclopentane-acetic acid; methyl 1-aminomethyl-1-cyclohexane-acetate; n-butyl 1-aminomethyl-1-cyclohexane-acetate; methyl 1-aminomethyl-1-cyclohexane-acetate; n-butyl 1-aminomethyl-1-cycloheptane acetate toluene sulfonate; 1-aminomethyl-1-cyclopentane-acetate benzenesulfonate; and n-butyl 1-aminomethyl-1-cyclopentane-acetate.
4. The delivery system of 1-3 further including an excipient.
5. The delivery system of 4 wherein said excipient is selected from the group consisting of carbohydrate materials, polyhydric alcohols, and mixtures thereof.
6. The delivery system of 4 wherein said excipient is selected from the group consisting of monosaccharides, disaccharides, polysaccharides, partially hydrolysed starch, corn syrup, sugar alcohols, and mixtures thereof.
7. The delivery system of 4 wherein said excipient is present in an amount of up to about 80% by dry weight of said core material.
8. The delivery system of 1 wherein said first coating comprises a water-insoluble film forming polymeric

material.

9. The delivery system of 8 wherein said water-insoluble film forming polymeric material is selected from the group consisting of acrylic acid and substituted acrylic acid polymers and copolymers; polysulfonic acid ester polymers; vinyl and substituted vinyl ester polymers and copolymers; cellulose ethers, their polymers and copolymers.

10. The delivery system of 8 wherein said water-insoluble film-forming polymeric material is selected from the group consisting of ethylcellulose; cellulose acetate; cellulose acetate phthalate; cellulose acetate butyrate; ethylene-vinyl acetates and/or phthalates; polyvinyl alcohol; polyvinyl acetates and/or phthalates; and ethyl and/or methyl methacrylic acids, esters, and copolymers.

11. The delivery system of 8 wherein said water-insoluble film-forming polymeric material is selected from the group consisting of ethylcellulose; an anionic copolymer based on polymethacrylic and acrylic acid ester; a neutral copolymer based on poly(meth)acrylic acid esters; and, a cationic copolymer based on dimethylaminoethyl methacrylate and neutral methacrylic acid esters; and mixtures thereof.

12. The delivery system of 1 wherein said first coating comprises a water-soluble film-forming polymeric material.

13. The delivery system of 1 wherein said first coating comprises a hydrocolloid.

14. The delivery system of 13 wherein said hydrocolloid is selected from the group consisting of gums, pectins, alginates, mucilages, and mixtures thereof.

15. The delivery system of 13 wherein said hydrocolloid is selected from the group consisting of gum arabic, tragacanth, karaya, ghatti, agar, alginates, carrageenans, fucellan, psyllium, and mixtures thereof.

16. The delivery system of 13 wherein said hydrocolloid is selected from the group consisting of polyvinyl pyrrolidone, gelatin, dextran, xanthan, curdan, cellulose, methylcellulose, hydroxyethyl cellulose, hydroxypropyl cellulose, hydroxypropyl methylcellulose, carboxymethyl cellulose, low methoxy pectin, propylene glycol alginate, and mixtures thereof.

17. The delivery system of 1 wherein said first coating is present in an amount of from about 10% to about 25% by dry weight.

18. The delivery system of 1 wherein said second coating is present in an amount of from about 20% to about 100% by dry weight.

19. The delivery system of 1 wherein said first coating is present in an amount of 25% by dry weight, and said second coating is present in an amount of 50% by dry weight.

20. The delivery system of 1 wherein the fat material is a fatty acid selected from the group consisting of hydrogenated or partially hydrogenated oils.

21. The delivery system of 20 wherein the hydrogenated or partially hydrogenated oils are selected from the group consisting of palm oil, palm kernel oil, soybean oil, rapeseed oil, rice bran oil, cottonseed oil, sunflower oil, safflower oil, and mixtures thereof.

22. The delivery system of 1 wherein the fat material is selected from the group consisting of monoglycerides, diglycerides, triglycerides, polyglycerol esters, sorbitol esters, and mixtures thereof.

23. The delivery system of 1 wherein the wax material is selected from the group consisting of natural waxes, synthetic waxes and mixtures thereof.

24. The delivery system of 23 wherein the wax material is selected from the group consisting of paraffin wax, beeswax, carnauba wax, candelilla wax, lanolin wax, bayberry wax, sugar cane wax, petrolatum, carbowax, spermaceti wax, rice bran wax, microcrystalline wax, and mixtures thereof.

25. The delivery system of 1 incorporated into a chewing gum composition.

26. The delivery system of 1 incorporated into a confectionery composition.

27. The delivery system of 1 incorporated into a pharmaceutical composition.

28. The delivery system of 1 prepared into a chewable tablet.

## Claims

1. A delivery system for a cyclic amino acid compound providing reduced bitterness with improved mouthfeel, compressibility, and high temperature stability, comprising:

    (a) a core material comprising a cyclic amino acid compound;

    (b) a first polymeric coating selected from water-insoluble and water-soluble polymeric film-forming materials, in an amount of from about 5% to about 100% by dry weight of the core material; and

    (c) a second hydrophilic coating selected from the group consisting of fats, fatty acids, waxes and mixtures thereof, present in an amount ranging from about 20% to about 400% by dry weight of the combination of said core material and said first hydrophilic coating.

2. The delivery system of Claim 1 wherein said cyclic amino acid compound has the formula:

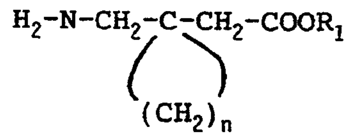

wherein $R_1$ is a hydrogen atom or a lower alkyl radical and n is 4, 5 or 6; and the pharmacologically compatible salt thereof.

3. The delivery system of either of claims 1 or 2, wherein said cyclic amino acid compound is selected from the group consisting of 1-aminomethyl-1-cyclohexane-acetic acid; ethyl-1-aminomethyl-1-cyclohexane-acetate; 1-aminomethyl-1-cycloheptane-acetic acid; 1-aminomethyl-1-cyclopentane-acetic acid; methyl 1-aminomethyl-1-cyclohexane-acetate; n-butyl 1-aminomethyl-1-cyclohexane-acetate; methyl 1-aminomethyl-1-cyclohexane-acetate; n-butyl 1-aminomethyl-1-cycloheptane acetate toluene sulfonate; 1-aminomethyl-1-cyclopentaneacetate benzene-sulfonate; and n-butyl 1-aminomethyl-1-cyclopentane-acetate.

4. The delivery system of any one of Claims 1-3, further including an excipient selected from the group consisting of carbohydrate materials, particularly monosaccharides, disaccharides, polysaccharides, partially hydrolysed starch, corn syrup, sugar alcohols, and mixtures thereof, and polyhydric alcohols, and mixtures thereof.

5. The delivery system of Claim 4, wherein said excipient is present in an amount of up to about 80% by dry weight of said core material.

6. The delivery system of any preceding Claim, wherein said first coating comprises a water-insoluble film forming polymeric material selected from the group consisting of acrylic acid and substituted acrylic acid polymers and copolymers; polysulfonic acid ester polymers; vinyl and substituted vinyl ester polymers and copolymers; cellulose ethers, their polymers and copolymers, ethylcellulose; cellulose acetate; cellulose acetate phthalate; cellulose acetate butyrate; ethylene-vinyl acetates and/or phthalates; polyvinyl alcohol; polyvinyl acetates and/or phthalates; and ethyl and/or methyl methacrylic acids, esters, and copolymers, ethylcellulose; an anionic copolymer based on polymethacrylic and acrylic acid ester; a neutral copolymer based on poly(meth)acrylic acid esters; and, a cationic copolymer based on dimethylaminoethyl methacrylate and neutral methacrylic acid esters; and mixtures thereof.

7. The delivery system of any one of Claims 1-5, wherein said first coating comprises a water-soluble film-forming polymeric material.

8. The delivery system of Claim 1, wherein said first coating comprises a hydrocolloid selected from the group consisting of gums, pectins, alginates, mucilages, and mixtures thereof, preferably gum arabic, tragacanth, karaya, ghatti, agar, alginates, carrageenans, fucellan, psyllium, and mixtures thereof, and polyvinyl pyrrolidone, gelatin, dextran, xanthan, curdan, cellulose, methylcellulose, hydroxyethyl cellulose, hydroxypropyl cellulose, hydroxypropyl methylcellulose, carboxymethyl cellulose, low methoxy pectin, propylene glycol alginate, and mixtures thereof.

9. The delivery system of any preceding Claim, wherein said first coating is present in an amount of from about 10% to about 25% by dry weight, and wherein said second coating is present in an amount of from about 20% to about 100% by dry weight.

10. The delivery system of any preceding Claim, wherein said first coating is present in an amount of 25% by dry weight, and said second coating is present in an amount of 50% by dry weight.

11. The delivery system of any preceding Claim, wherein the fat material is:
a) a fatty acid comprised by hydrogenated or partially hydrogenated oils which are selected from the group consisting of palm oil, palm kernel oil, soybean oil, rapeseed oil, rice bran oil, cottonseed oil, sunflower oil, safflower oil, and mixtures thereof, or

b) a material selected from the group consisting of monoglycerides, diglycerides, triglycerides, polyglycerol esters, sorbitol esters, and mixtures thereof.

**12.** The delivery system of any preceding Claim, wherein the wax material is selected from the group consisting of natural waxes, synthetic waxes and mixtures thereof, preferably paraffin wax, beeswax, carnauba wax, candelilla wax, lanolin wax, bayberry wax, sugar cane wax, petrolatum, carbowax, spermaceti wax, rice bran wax, microcrystalline wax, and mixtures thereof.

**13.** The delivery system of any preceding Claim, when incorporated into a composition selected from the group consisting of chewing gum compositions, confectionery compositions, pharmaceutical compositions, and chewable tablets.

European Patent Office

# EUROPEAN SEARCH REPORT

Application Number

EP 91 81 0380

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 340 677 (WARNER LAMBERT CO.)<br>* Claims 1,11; page 4, lines 55-56; page 5, lines 1-12 *<br>--- | 1-4,7-11,13 | A 61 K 31/195<br>A 61 K 9/54<br>A 61 K 31/215 |
| A | WO-A-8 905 635 (SHINETSU CHEMICAL CO., LTD)<br>* Claims 1-3; page 5, lines 5-19; page 7, lines 16-17; page 15, example 2; page 25, lines 10-13 *<br>--- | 1,4,6,9,11-13 | |
| A | EP-A-0 148 811 (LEJUS MEDICAL AB)<br>* Claim 1; page 2, line 37 - page 3, line 2; page 3, lines 10-37; page 4, lines 8-14 *<br>----- | 1,4,6,11-13 | |
| | | | TECHNICAL FIELDS SEARCHED (Int. Cl.5) |
| | | | A 61 K |

The present search report has been drawn up for all claims

| Place of search | Date of completion of the search | Examiner |
|---|---|---|
| THE HAGUE | 21-08-1991 | VENTURA AMAT A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)